# EUROPÄISCHE PATENTANMELDUNG

(11) **EP 3 643 291 A1**
(43) Veröffentlichungstag der Anmeldung: **29.04.2020**
(21) Anmeldenummer: 19201425.6
(22) Anmeldetag: 04.10.2019
(51) Int. Cl.: A61K 8/19, A61K 8/9789, A61Q 11/00

(54) **MUND- UND/ODER ZAHNPFLEGE- UND/ODER -REINIGUNGSMITTEL MIT MINERALSTOFFEN**

(30) Priorität: 24.10.2018 DE 102018126513; 13.03.2019 DE 102019106444
(71) Anmelder: Rösch, Britta, 09432 Grossolbersdorf (DE)
(72) Erfinder: Rösch, Britta, 09432 Grossolbersdorf (DE)
(74) Vertreter: Lohmanns, Bernard

(57) **Zusammenfassung**

Die Anmeldung beschreibt ein Mund- und/oder Zahnpflege- und/oder -reinigungsmittel, enthaltend in einem oral akzeptablen Träger mindestens drei Mineralstoffe pflanzlichen Ursprungs zur Remineralisierung der Zähne, des Zahnfleisches, der Lippen und/oder der Mundschleimhaut.

## Beschreibung

Die Erfindung betrifft ein Mittel zur Mund- und/oder Zahnpflege- und/oder -reinigung, das Mineralstoffe enthält. Die Erfindung betrifft auch ein Verfahren zur Herstellung eines solchen Mund- und/oder Zahnpflege- und/oder -reinigungsmittels und ein Verfahren zur Reinigung und/oder Pflege des Mundes und/oder der Zähne mit dem Mittel.

Zahnpflege- und -reinigungsmittel sind in verschiedenen Formen auf dem Markt und dienen in erster Linie der Reinigung der Zahnoberfläche und der Vorbeugung von Zahn- und Zahnfleischerkrankungen. Zahnpflege- und/oder -reinigungsmittel werden vor allem in Form von Pasten, Cremes oder Gelen angeboten. In den letzten Jahren haben auch Liquid- oder Flüssigzahncremes und Mundwässer zunehmend an Bedeutung gewonnen.

In den letzten Jahren konnte eine Zunahme von Defekten an Zahnschmelz und Zahnbein durch dentale Erosionen beobachtet werden. Dentale Erosionen oder Zahnerosionen sind irreversible Schädigungen der Zahnhartsubstanz durch Säuren aus Lebensmitteln (beispielsweise Obst) und Getränken (beispielsweise Softdrinks). Die Säuren führen zu einer Entmineralisierung der Zahnhartsubstanz und weichen dadurch zuerst den Zahnschmelz und dann das Zahnbein (Dentin) auf.

Neben der täglichen Mundhygiene fördern auch Vitamine und Mineralstoffe die Mundgesundheit. So helfen insbesondere Calcium und Phosphat den Zahnschmelz zu härten und vor Angriffen durch säure- und zuckerhaltige Speisen und Getränke zu schützen. Magnesium beugt Zahnfleischentzündungen und der altersbedingten Zunahme von Parodontitis vor.

Mineralstoffe sind lebensnotwendige anorganische Nährstoffe, welche der Organismus nicht selbst herstellen kann. Sie müssen ihm beispielsweise mit der Nahrung zugeführt werden. Mineralstoffe werden in zwei Gruppen eingeteilt: Elemente mit einer höheren Konzentration als 50 mg pro kg Körpergewicht werden als *Mengen- oder Makroelemente* bezeichnet. Elemente mit weniger als 50 mg pro kg Körpergewicht heißen *Spuren- oder Mikroelemente.* Eine Ausnahme bildet Eisen, es zählt zwar zu den Spurenelementen, liegt jedoch mit ungefähr 60 mg pro kg Körpergewicht über der Definition. Als essentielle Spuren- oder Mikroelemente werden Spuren- oder Mikroelemente bezeichnet, die für einen Menschen lebensnotwendig sind.

Nicht immer reicht eine vielseitige Ernährung aus, um den Bedarf an Vitaminen und/oder Mineralstoffen zu decken. Mit Hilfe von Nahrungsergänzungsmitteln kann der Vitamin- und/oder Mineralstoffspiegel im Körper erhöht werden.

Im Handel sind remineralisierende Zahncremes und -pasten erhältlich. Diese enthalten neben Fluorid beispielsweise Hydroxylapatit. Hydroxylapatit ist ein hydroxyliertes Calciumphosphatsalz und bildet die Grundlage der Hartsubstanz aller Wirbeltiere. Hydroxylapatit ist im Zahnbein (Dentin) zu 70 % und im Zahnschmelz (Enamelum) zu 95 % enthalten.

Die Verfügbarkeit von Calcium-Verbindungen für die erwünschte Remineralisierung hängt ganz entscheidend von der Teilchengroße dieser in Wasser schwerlöslichen und in den Zahnpflege- und -reinigungsmitteln dispergierten Komponenten ab. In der EP 786 245 A1 werden Zahnpflegemittel beschrieben, die Hydroxylapatitpartikel mit Partikelgrößen von 0,05 bis 1,0 µm umfassen, die durch Mahlen erhalten werden.

Es besteht weiterhin Bedarf an remineralisierenden Mund- und/oder Zahnpflege- und/oder -reinigung, die die remineralisierende Verbindung mit einer hohen Verfügbarkeit für den menschlichen Körper enthalten.

Diese Aufgabe wird gelöst durch ein Mund- und/oder Zahnpflege- und/oder -reinigungsmittel, enthaltend in einem oral akzeptablen Träger mindestens drei Mineralstoffe pflanzlichen Ursprungs.

Überraschenderweise hat sich gezeigt, dass Mineralstoffe pflanzlichen Ursprungs vom menschlichen Körper, insbesondere auch von den Zähnen, der Mundschleimhaut, den Lippen und/oder dem Zahnfleisch effektiver aufgenommen werden, als Mineralstoffe geologischen oder marinen Ursprungs. Mineralstoffe pflanzlichen Ursprungs werden auch als Phyto-Mineralstoffe bezeichnet.

Die Mineralstoffe pflanzlichen Ursprungs liegen vorzugsweise in kolloidaler Form und/oder in komplexierter Form vor.

Kolloide sind extrem fein verteilte und sehr kleine Teilchen, die sich in Wasser nicht lösen und frei beweglich sind. Mineralstoffe pflanzlichen Ursprungs in kolloidaler Form liegen frei ionisiert und damit in biologisch aktiver Form vor. Mineralstoffe pflanzlichen Ursprungs in kolloidaler Form weisen vorzugsweise eine Teilchengröße ≤ 1.000 nm auf.

Mineralstoffe pflanzlichen Ursprungs in komplexierter Form umfassen einen metallischen Mineralstoff als Zentralatom und organische und/oder anorganische Verbindungen als Liganden. Ein Ligand ist vorzugsweise mit dem Zentralatom über mindestens zwei Stellen/Positionen verbunden. In einer besonders bevorzugten Ausführungsform umfassen die Mineralstoffe pflanzlichen Ursprungs in komplexierter Form organische Verbindungen als Liganden. Bevorzugte organische Liganden umfassen Citrat, Lactat, Gluconat, Orotat, Ascorbat, Acetat, Aspartat, Glutamat, Glycerophosphat, Pidolat und Mischungen daraus.

In einer besonders bevorzugten Ausführungsform liegen die Mineralstoffe pflanzlichen Ursprungs in kolloidaler Form und in komplexierter Form mit organischen Verbindungen als Liganden vor.

Durch das Vorliegen in kolloidaler Form und in komplexierter Form mit organischen Verbindungen weisen die Mineralstoffe pflanzlichen Ursprungs eine hohe Bioaktivität und eine hohe Bioverfügbarkeit auf.

Bevorzugte Mineralstoffe pflanzlichen Ursprungs umfassen Arsen (As), Bor (B), Calcium (Ca), Chrom (Cr), Kupfer (Cu), Eisen (Fe), Magnesium (Mg), Mangan (Mn), Chlorid, Cobalt (Co), Bor (B), Phosphor (P), Schwefel (S), Iod (I), Antimon (Sb), Barium (Ba), Beryllium (Be), Bismut (Bi), Cer (Ce), Cäsium (Cs), Dysprosium (Dy), Erbium (Er), Europium (Eu), Fluor (F), Gadolinium (Gd), Gallium (Ga), Germanium (Ge), Gold (Au), Hafnium (Hf), Holmium (Ho), Iridium (Ir), Kalium (K), Lanthan (La), Lithium (Li), Lutetium (Lu), Magnesium (Mg), Mangan (Mn), Molybdän (Mo), Natrium (Na), Neodym (Nd), Nickel (Ni), Niob (Nb), Osmium (Os), Palladium (Pd), Phosphor (P), Platin (Pt), Praseodym (Pr), Rhenium (Rh), Rhodium (Rb), Rubidium (Rb), Ruthenium (Ru), Samarium (Sm), Scandium (Sc), Schwefel (S), Seien (Se), Silber (Ag), Silicium (Si), Strontium (Sr), Tellur (Te), Terbium (Tb), Thallium (Tl), Thorium (Th), Zinn (Sn), Titan (Ti), Wolfram (W), Vanadium (V), Ytterbium (Yb), Yttrium (Y), Zink (Zn), Zinn (Sn) und/oder Zirkonium (Zr).

Ganz besonders bevorzugte Mineralstoffe pflanzlichen Ursprungs umfassen Calcium (Ca), Chlorid, Kalium (K), Magnesium (Mg), Natrium (Na), Phosphor (P), Schwefel (S), Cobalt (Co), Eisen (Fe), Fluor (F), Iod (I), Kupfer (Cu), Mangan (Mn), Molybdän (Mo), Selen (Se), Silicium (Si), Vanadium (V), Zink (Zn), Chrom (Cr), Arsen (As), Bor (B), Rubidium (Rb) und/oder Zinn (Sn).

Es ist bevorzugt, dass das Mittel mindestens fünf, mehr bevorzugt mindestens acht, Mineralstoffe pflanzlichen Ursprungs enthält. In einer ganz besonders bevorzugten Ausführungsform sind die mindestens fünf, vorzugsweise mindestens acht, Mineralstoffe pflanzlichen Ursprungs ausgewählt aus der Gruppe bestehend Calcium (Ca), Chrom (Cr), Kupfer (Cu), Eisen (Fe), Magnesium (Mg), Mangan (Mn), Selen (Se), Zink (Zn) und Mischungen daraus.

Der Begriff "pflanzlichen Ursprungs" bedeutet, dass die Mineralstoffe aus Pflanzen gewonnen wurden. Pflanzen im Sinne dieser Anmeldung sind Organismen, die zur Gruppe der Eukaryonten zählen. Algen bezeichnen im Wasser oder feuchten Gebieten lebende, eukaryotische, pflanzenartige Lebewesen, die Photosynthese betreiben, jedoch nicht zu den eigentlichen Pflanzen gehören. Entsprechend umfasst ein Mineralstoff pflanzlichen Ursprungs keinen Mineralstoff, der aus Algen gewonnen wurde.

Mischungen von Mineralstoffen pflanzlichen Ursprungs in Form von wässrigen Lösungen sind kommerziell beispielsweise unter der Bezeichnung "Mikromineralien Ur-Essenz" von Rocky Mountain Phyto Essentials erhältlich.

Neben den oben genannten, meist metallischen, Elementen, die in kolloidaler Form und/oder in komplexierter Form vorliegen, können die Mischungen von Mineralstoffen pflanzlichen Ursprungs in Form einer wässrigen Lösung weitere Inhaltsstoffe, die vorzugsweise Chlorid, Sulfat, Nitrat, Fluorid, Hydrogencarbonat und/oder Fulvosäuren umfassen, enthalten.

In einer ganz besonders bevorzugten Ausführungsform des Mittels, enthält dieses ferner Chlorid, Sulfat, Nitrat, Fluorid, Hydrogencarbonat und/oder Fulvosäuren als weitere Inhaltsstoffe.

Das Mittel weist einen oral akzeptablen Träger auf. Der Träger kann flüssig oder fest, beispielsweise pulverförmig, sein. Ein bevorzugter oral akzeptabler Träger ist flüssig und kann Wasser und/oder einen oder mehr mehrwertige Alkohole wie beispielsweise Glycerin, Sorbit oder ein Alkylenglycol wie Polyethylenglycol oder Propylenglycol umfassen. Vorzugsweise enthält der oral akzeptable Träger Wasser und Glycerin. Das Glycerin ist bevorzugt in einer Menge von mindestens 30 Gew.-%, mehr bevorzugt von mindestens 40 Gew.-% und ganz besonders bevorzugt von mindestens 50 Gew.-%, jeweils bezogen auf das Gesamtgewicht des Mittels.

Bevorzugte Mittel sind dadurch gekennzeichnet, dass sie ferner mindestens ein pflanzliches Öl enthalten.

Das pflanzliche Öl bleibt als Beschichtung auf den damit behandelten Oberflächen, insbesondere Zahnoberflächen, zurück und schützt diese vor nachfolgenden Säureeinflüssen. Die Beschichtung mit dem pflanzlichen Öl bewirkt, dass auf den behandelten Oberflächen länger ein basischer pH aufrecht erhalten wird.

In einer bevorzugten Ausführungsform ist das mindestens eine pflanzliche Öl ausgewählt aus der Gruppe bestehend aus der Gruppe Amaranthsamenöl, Arganöl, Reiskeimöl, Baobab-Öl, Manetti-Öl, Marulasamenöl, Yangusamen-Öl, Rambutan-Öl, Buckthorn-Öl, Monoi de Tahiti, Tigernut-Öl, Inca Inchi-Öl, Avocadoöl, Baumwollsamenöl, Cupuaçu-Butter, Cashewöl, Distelöl, Erdnussöl, Jojobaöl, Kamilleöl, Kokosöl, Kokosnusskernbutter, Kürbiskernöl, Leinöl, Macadamiaöl, Maiskeimöl, Mandelöl, Aprikosenkernöl, Mohnöl, Nachtkerzenöl, Olivenöl, Rapsöl, Sojaöl, Sonnenblumenöl und Weizenkeimöl, insbesondere (-)-α-Bisabolol, hydriertes Jojobaöl und Mischungen daraus.

In einer besonders bevorzugten Ausführungsform umfasst das mindestens eine pflanzliche Öl Kokosöl.

Kokosöl wird aus der Steinfrucht der Kokospalme aus der Familie der *Arecaceae* (Palmengewächse), exakter aus »Kopra« gewonnen, dem zerkleinerten und getrockneten Nährgewebe (Fruchtfleisch) der Kokosnuss, das getrocknet ca. 70 % Fett enthält. Kokosöl weist einen Schmelzpunkt im Bereich von 23 bis 26 °C auf. Bei Kontakt mit der Haut, den Lippen, der Mundschleimhaut und/oder dem Zahnfleisch schmilzt das Kokosöl und bewirkt durch die aufgenommene Wärme beim Schmelzen einen leicht kühlenden Effekt. Der leicht kühlende Effekt bewirkt bei den Anwendern nach Anwendung des Mittels einen Frische-Effekt.

Die Menge an dem mindestens einem pflanzlichen Öl, vorzugsweise dem Kokosöl, in dem Mittel beträgt bevorzugt 1 bis 10 Gew.-%, mehr bevorzugt 1 bis 9 Gew.-% und besonders bevorzugt 3 bis 8 Gew.-%, jeweils bezogen auf das Gesamtgewicht des Mittels.

In einer bevorzugten Ausführungsform enthält das Mittel ferner mindestens einen Pflanzenextrakt.

Ein Pflanzenextrakt ist eine Zubereitung von flüssiger, halbfester oder fester Beschaffenheit, die aus üblicherweise getrockneten Pflanzen und/oder Pflanzenteilen hergestellt wurde.

Die Herstellung des mindestens einen Pflanzenextraktes kann in jeder dem Fachmann bekannten Weise unter Verwendung jedes beliebigen Pflanzengewebes und unter Verwendung jedes beliebigen Extraktionsmittels erfolgen. So kann der Pflanzenextrakt beispielsweise durch Extraktion der gesamten Pflanze, durch Extraktion aus Blüten, Früchten, Rinde, Blättern, Samen, Kerne, Wurzeln und/oder durch Extraktion aus dem Meristem der Pflanze erfolgen.

Als Extraktionsmittel zur Herstellung des mindestens einen Pflanzenextrakts können beispielsweise Wasser, Alkohole, Öle, organische Lösungsmittel sowie deren Mischungen verwendet werden. Als Alkohole kommen beispielsweise niedere Alkohole wie Ethanol und Isopropanol, insbesondere aber auch mehrwertige Alkohole wie Ethylenglycol, Propylenglycol, Butylenglycol und insbesondere Glycerin in Frage, und zwar sowohl als alleiniges Extraktionsmittel als auch in Mischung mit Wasser. Es ist ganz besonders bevorzugt, dass das Extraktionsmittel Glycerin oder eine Mischung aus Wasser und Glycerin, wobei das Mischungsverhältnis vorzugsweise im Bereich von 1 : 1 bis 1 : 7 liegt, umfasst. Die Extraktion der Pflanzen und/oder Pflanzenbestandteile erfolgt auf übliche Weise, beispielsweise durch Mazeration, Perkolation, Reperkolation, Ultraschallextraktion, Digestion, Gegenstromextraktion, Soxhlet-Extraktion, Diakolation oder Kettenperkolation. Insbesondere bevorzugt erfolgt die Extraktion mittels Mazeration. Es ist weiter bevorzugt, dass vor der Mazeration eine Homogenisierung und/oder Erwärmung der zu extrahierenden Pflanzen und/oder Pflanzenbestandteile in dem Extraktionsmittel stattfindet.

In einer bevorzugten Ausführungsform ist der mindestens eine Pflanzenextrakt ausgewählt aus der Gruppe bestehend aus Kamillen-Extrakt (Matricaria chamomilla, Chamomilla recutita), Ingwer-Extrakt (Zingiber officinale), Cistacea-Extrakt (Cistus incanus, Cistus creticus, Cistus villosus creticus, Cistus ladanifer), Grüntee-Extrakt (Camellia sinensis, Camellia assamica), Löwenzahn-Extrakt (Taraxacum officinale), Galgant-Extrakt (Alpinia officinarum), Schafgarben-Extrakt (Achillea millefolium), Oregano-Extrakt (Origanum vulgare), Römischer Bertram-Extrakt (Anacyclus pyrethrum), Deutscher Bertram-Extrakt (Anacyclus officinarum), Echter Salbei-Extrakt (Salvia Officinalis), Aloe Vera-Extrakt (Aloe vera, Aloe barbadensis, Aloe perfoliata, Aloe vulgaris, Aloe indica, Aloe chinensis), Teebaum-Extrakt (Melaleuca alternifolia), Roßkastanien-Extrakt (Aesculus hippocastanum), Pfefferminz-Extrakt (Mentha piperita), Neem-Extrakt (Azadirachta indica), Süßholz (Glycyrrhiza glabra), Gelbholz-Extrakt (Xanthoxylum fraxineum), Thymian-Extrakt (Thymus vulgaris), Gingko-Extrakt (Ginkgo biloba), Zimt-Extrakt (Cinnamomum zeylanicum), Fenchel-Extrakt (Foeniculum vulgare), Eucalyptus-Extrakt (Eucalyptus globulus), Schachtelhalm-Extrakt (Equisetum arvense), Myrrhe-Extrakt (Commiphora molmol, Commiphora Abyssinica), Sonnenhut-Extrakt (Echinacea purpurea, Echinacea angustifolia, Echinacea pallida), Blutwurz (Potentilla tormentilla), Heidelbeer-Extrakt (Vaccinium myrtillus), Ringelblumen-Extrakt (Calendula officinalis), Hamamelis-Extrakt (Hamamelis virginiana), Olivenbaum-Extrakt (Olea europaea), Stevia-Extrakt (Stevia rebaudiana), Beinwell-Extrakt (Symphytum officinale), Maulbeerbaum-Extrakt (Morus niger) und Mischungen daraus.

In einer besonders bevorzugten Ausführungsform ist der Pflanzenextrakt ausgewählt aus der Gruppe bestehend aus Löwenzahn-Extrakt (Taraxacum officinale), Galgant-Extrakt (Alpinia officinarum), Schafgarben-Extrakt (Achillea millefolium), Oregano-Extrakt (Origanum vulgare) und Mischungen daraus.

Es ist bevorzugt, dass das Mittel mindestens zwei, mehr bevorzugt mindestens drei und noch mehr bevorzugt mindestens vier Pflanzenextrakte enthält.

In einer ganz besonders bevorzugten Ausführungsform ist das Mittel dadurch gekennzeichnet, dass es Löwenzahn-Extrakt (Taraxacum officinale), Galgant-Extrakt (Alpinia officinarum), Schafgarben-Extrakt (Achillea millefolium) und Oregano-Extrakt (Origanum vulgare) enthält.

Der Löwenzahn-Extrakt wird vorzugsweise aus der ganzen Pflanze, der Galgant-Extrakt aus der Wurzel, der Schafgarben-Extrakt aus dem Kraut und der Oregano-Extrakt aus dem Kraut hergestellt.

Die Kombination dieser Pflanzenextrakte führt zu einem selbst-konservierenden Mittel und es kann auf den Einsatz von Konservierungsmittel, wie zum Beispiel Parabene oder Hydroxybenzoesäureester, verzichtet werden. Außerdem wirken sich die Pflanzenextrakte aufgrund der Heilwirkung ihrer Inhaltsstoffe positiv auf die Gesundheit des Anwenders, beispielsweise auf die Gesundheit des Zahnfleischs, der Zähne, der Mundschleimhaut, der Zunge und/oder der Lippen, aus.

Vorzugsweise erfolgt die Herstellung der eingesetzten Pflanzenextrakte mittels Mazeration einer Mischung, umfassend a) von 1 bis 5 Gew.-% einer Pflanze und/oder eines Pflanzenbestandteils und b) 95 bis 99 Gew,-% einer Mischung aus Wasser und Glycerin, wobei das Mischungsverhältnis vorzugsweise im Bereich von 1 : 1 bis 1 : 7 liegt. Die Dauer der Mazeration liegt vorzugsweise zwischen 6 und 36 Stunden und mehr bevorzugt zwischen 12 und 24 Stunden. Nach der Mazeration werden die extrahierten Pflanzen und/oder die extrahierten Pflanzenbestandteile mittels Filtration entfernt und der Pflanzenextrakt erhalten. Es kann auch bevorzugt sein, dass die nach der Filtration erhaltenen Pflanzenextrakte weiteren üblichen Aufbereitungsschritten, wie beispielsweise Konzentration und/oder Entfärbung, unterzogen werden. Es ist weiter bevorzugt, dass die Mischung vor der Mazeration homogenisiert und/oder erhitzt wird. Es kann ferner bevorzugt sein, dass die Pflanzen und/oder die Pflanzenbestandteile vor der Mazeration getrocknet und mechanisch zerkleinert werden.

Alternativ oder zusätzlich zu dem mindestens einen Pflanzenextrakt kann das Mittel ferner mindestens eine Pflanze und/oder mindestens einen Pflanzenbestandteil in zerkleinerter Form enthalten.

Die mindestens eine Pflanze und/oder der mindestens einen Pflanzenbestandteil können in zerkleinerter Form entweder unmittelbar in frischer oder getrockneter Form in das Mittel eingearbeitet werden. Bevorzugt wird die mindestens eine Pflanze und/oder der mindestens eine Pflanzenbestandteil unmittelbar in getrockneter, zerkleinerter Form dem Mittel zugesetzt.

Die Trocknung der Pflanze oder des Bestandteils/der Bestandteile der Pflanze kann durch eine der bekannten Trocknungsarten erfolgen, wobei Lufttrocknung oder Gefriertrocknung bevorzugt sind.

Die Zerkleinerung der Pflanze und/oder des Pflanzenbestandteils kann durch jedes geeignete Verfahren erfolgen, wie zum Beispiel Zerreiben, Zermahlen, Zerschneiden oder Zerhäckseln. Nach dem Zerkleinern kann das hergestellte Pulver oder Mus durch geeignete Maßnahmen in Größenfraktionen getrennt werden, z.B. durch Sieben (insbesondere bei Pulver), Aufschlämmen (bei Mus) usw.

In einer bevorzugten Ausführungsform ist die mindestens eine Pflanze und/oder der mindestens eine Pflanzenbestandteil aus Pflanzen und/oder mindestens eines derer Bestandteile ausgewählt aus der Gruppe bestehend aus Kamille (Matricaria chamomilla, Chamomilla recutita), Ingwer (Zingiber officinale), Cistacea (Cistus incanus, Cistus creticus, Cistus villosus creticus, Cistus ladanifer), Grüntee (Camellia sinensis, Camellia assamica), Löwenzahn (Taraxacum officinale), Galgant (Alpinia officinarum), Schafgarben (Achillea millefolium), Oregano (Origanum vulgare), Deutscher Bertram (Anacyclus officinarum), Römischer Bertram (Anacyclus pyrethrum), Echter Salbei (Salvia Officinalis), Aloe Vera (Aloe vera, Aloe barbadensis, Aloe perfoliata, Aloe vulgaris, Aloe indica, Aloe chinensis), Teebaum (Melaleuca alternifolia), Roßkastanie (Aesculus hippocastanum), Pfefferminze (Mentha piperita), Neem (Azadirachta indica), Süßholz (Glycyrrhiza glabra), Gelbholz (Xanthoxylum fraxineum), Thymian (Thymus vulgaris), Gingko (Ginkgo biloba), Zimt (Cinnamomum zeylanicum), Fenchel (Foeniculum vulgare), Eucalyptus (Eucalyptus globulus), Schachtelhalm (Equisetum arvense), Myrrhe (Commiphora molmol, Commiphora Abyssinica), Sonnenhut (Echinacea purpurea, Echinacea angustifolia, Echinacea pallida), Blutwurz (Potentilla tormentilla), Heidelbeer (Vaccinium myrtillus), Ringelblumen (Calendula officinalis), Hamamelis (Hamamelis virginiana), Olivenbaum (Olea europaea), Stevia (Stevia rebaudiana), Beinwell (Symphytum officinale), Maulbeerbaum (Morus niger) und Mischungen daraus.

Der mindestens eine Pflanzenbestandteil kann ausgewählt sein aus der Gruppe bestehend aus Blüten, Früchten, Rinde, Blättern, Samen, Kerne, Wurzeln und Mischungen daraus.

Es ist äußerst bevorzugt, dass die mindestens eine Pflanze und/oder der mindestens eine Pflanzenbestandteil in zerkleinerter Form aus der Pflanzengattung Bertram (Anacyclus), auch Ringblumen oder Ringkörbchen genannt, stammt. Es gibt 8 bis 13 Anacyclus-Arten, von denen der Deutsche Bertram (Anacyclus officinarum) und der Römische Bertram (Anacyclus pyrethrum) bevorzugt sind. Es ist insbesondere bevorzugt, dass die mindestens eine Pflanze und/oder der mindestens eine Pflanzenbestandteil in zerkleinerter Form zerkleinerte Bertramwurzel enthält. Es ist äußerst bevorzugt, dass die mindestens eine Pflanze und/oder der mindestens eine Pflanzenbestandteil in zerkleinerter Form zerkleinerte Wurzel des Deutschen Bertrams und/oder des Römischen Bertrams enthält.

In einer äußerst bevorzugten Ausführungsform ist das Mittel dadurch gekennzeichnet, dass es mindestens vier Pflanzenextrakte und mindestens einen Pflanzenbestandteil in zerkleinerter Form enthält. In dieser äußerst bevorzugten Ausführungsform umfasst das Mittel bevorzugt Löwenzahn-Extrakt (Taraxacum officinale), Galgant-Extrakt (Alpinia officinarum), Schafgarben-Extrakt (Achillea millefolium), Oregano-Extrakt (Origanum vulgare) und zerkleinerte Bertramwurzel.

Ein weiterer, optionaler Inhaltsstoff des Mund- und/oder Zahnpflege- und/oder - reinigungsmittels sind natürliche ätherische Öle, insbesondere Teebaum-Öl, Salbei-Öl, Pfefferminz-Öl, Rosmarinöl, Eukalyptusöl, Krauseminzöl, Anisöl, Fenchelöl, Kümmelöl und/oder Nelkenöl. Diese ätherischen Öle können die organoleptischen Eigenschaften der Mittel verbessern.

In einer weiteren, bevorzugten Ausführungsform der Erfindung umfasst das Mittel ferner mindestens ein Tonmineral.

Tonmineral bezeichnet einerseits Minerale, die überwiegend feinstkörnig (Korngröße < 2 µm) vorkommen, andererseits jedoch die Schichtsilikate, die nach ihrer schichtartigen Kristallstruktur aus Silizium und Sauerstoff, sowie Wasserstoff und meist Magnesium und Aluminium benannt sind. Beide Definitionen sind nicht deckungsgleich.

Es ist insbesondere bevorzugt, dass das Tonmineral ausgewählt ist aus der Gruppe bestehend aus der Kaolin-Gruppe, der Smektit-Gruppe, der Illit-Glimmer-Gruppe, der Chlorit-Gruppe und Mischungen daraus.

Es ist äußerst bevorzugt, dass das Tonmineral ein Schichtsilicat ausgewählt aus der Gruppe bestehend aus Kaolinit, dioktaedrischem Vermiculit, dioktaedrischem Illit, dioktaedrischem Smectit, Montmorillonit, Beidellit, Nontronit, Volkonskoit, Nakrit, trioktaedrischem Vermiculit, trioktaedrischer Smektit, Saponit, Hectorit, Saukonit und Mischungen daraus umfasst.

Tonmineralien können dem Mittel in Form von Tonerden zugesetzt werden. Es gibt verschiedene Tonerden, die sich durch eine unterschiedliche Zusammensetzung an Tonmineralien und ggf. Schichtsilikaten auszeichnen. Tonerde gibt es in weiß, rosa, rot-gelb, braun und grün. Die Farbe von grüner Tonerde, die beispielsweise die Schichtsilikate Illit und Montmorillonit enthält, ist auf Kupferverbindungen zurückzuführen. Bei rosafarbener, roter und brauner Tonerde ist Eisen das farbgebende Element. Geeignete grüne Tonerden sind kommerziell, beispielsweise unter den Bezeichnungen "Velay Green Clay" von Argile Verte du Velay erhältlich.

Das Mittel kann ein oder mehr Tonminerale und dadurch ein oder mehr Schichtsilicate umfassen. Es ist bevorzugt, dass das Mittel mindestens zwei oder drei Tonminerale enthält. Es ist insbesondere bevorzugt, dass das Mittel mindestens ein Tonmineral aus der Kaolin-Gruppe, mindestens ein Tonmineral aus der Smektit-Gruppe und mindestens ein Tonmineral der Illit-Glimmer-Gruppe enthält. Bevorzugt ist eine Kombination aus Illit, Kaolin und Montmorillonit. Diese Kombination findet sich beispielsweise in grüner Tonerde. Es kann deshalb bevorzugt sein, dass das Mund- und/oder Zahnpflege- und/oder -reinigungsmittel das mindestens eine Tonmineral in Form einer grünen Tonerde enthält.

Die Menge an Tonmineral beträgt vorzugsweise 10 bis 40 Gew.-%, mehr bevorzugt 15 bis 35 Gew.-% und ganz besonders bevorzugt 20 bis 30 Gew.-%, jeweils bezogen auf das Gesamtgewicht des Mittels,

Die Tonminerale dienen insbesondere als Poliermittel und als Lieferant für weitere Mineralstoffe. Die quellfähigen Schichtsilikate, wie Montmorillonit, eigenen sich auch zur Viskositätskontrolle/Konsistenzgebung.

Die Mittel können alternativ oder zusätzlich zu einem quellfähigen Schichtsilikat als Konsistenzregler oder Bindemittel natürliche und/oder synthetische wasserlösliche Polymere wie Alginate, Carragheenate, Traganth, Stärke und Stärkeether, Celluloseether wie z.B. Carboxymethylcellulose (Na-Salz), Hydroxyethylcellulose, Methylhydroxypropylcellulose, Guar, Akaziengum, Agar-Agar, Xanthan, Succinoglycan-Gum, Johannisbrotmehl, Pektine, wasserlösliche Carboxyvinylpolymere (z. B. Carbopol®-Typen), Polyvinylalkohol, Polyvinylpyrrolidon, Polyethylenglycole, insbesondere solche mit Molekulargewichten von 1.500-1.000.000, enthalten.

Weitere Stoffe, die sich als Konsistenzregler eignen sind kolloidale Verdickungskieselsäuren, pyrogene Kieselsäuren oder feinst vermahlene Fällungskieselsäuren. Es können auch viskositätsstabilisierende Zusätze aus der Gruppe der kationischen, zwitterionischen oder ampholytischen stickstoffhaltigen Tenside in dem Mund- und/oder Zahnpflege- und/oder -reinigungsmittel verwendet werden.

Als besonders verträglich mit den Mineralstoffen pflanzlichen Ursprungs haben sich Carboxymethylcellulose (Na-Salz) und insbesondere Xanthan erwiesen. Besonders bevorzugte Mund- und/oder Zahnpflege- und/oder -reinigungsmittel sind dadurch gekennzeichnet, dass sie - bezogen auf ihr Gesamtgewicht - 0,1 bis 5 Gew.-%, vorzugsweise 0,5 bis 4 Gew.-% und insbesondere bevorzugt 1 bis 3 Gew.-%, Xanthan enthalten.

Geeignete Xanthane sind beispielsweise unter der Bezeichnung Xanthural® von CP Kelco erhältlich.

In einer weiteren, ebenfalls bevorzugten Ausführungsform der Erfindung umfasst das Mittel ferner mindestens ein Gerüstsilikat. Gerüstsilikate (Tektosilikate) bezeichnet Silikate, deren Silikatanionen aus einem Gerüst eckenverknüpfter SiO₄- und AlO₄-Tetraeder bestehen. Es ist besonders bevorzugt, dass das Mittel einen natürlichen und/oder synthetischen Zeolith als Gerüstsilikat enthält.

Natürliche Zeolithe sind aus einer Umkristallisation vulkanischer Gläser entstanden und haben daher noch mehr oder weniger hohe Restbestandteile der ursprünglichen Phase. Man nennt diese feindispers gemischten Mineralien "Zeolithite": Ein "Naturzeolith", der z. B. aus 87% Klinoptilolith mit 13% anderer Silikatphasen besteht, ist ein Klinoptilolith-Zeolithit. Von den 60 natürlich vorkommenden Zeolithen steht insbesondere Klinoptilolith in kommerziell verwendbaren Mengen zur Verfügung. Weitere geeignete natürliche Zeolithe umfassen beispielsweise Chabasit, Stilbit und/oder Mordenit. In einer bevorzugten Ausführungsform umfasst das mindestens eine Gerüstsilikat Klinoptilolith.

Klinoptilolith ist die Sammelbezeichnung für eine Gruppe nicht näher spezifizierter Minerale bei denen es sich um die Endglieder einer lückenlosen Mischreihe mit folgenden idealisierten Zusammensetzungen handelt:
- Klinoptilolith-Ca: Ca₃(Si₃₀Al₆)O₇₂·20H₂O
- Klinoptilolith-K: K₆(Si₃₀Al₆)O₇₂·20H₂O
- Klinoptilolith-Na: Na₆(Si₃₀Al₆)O₇₂·20H₂O

In einer vorteilhaften Ausführungsform des Mittels wird das Gerüstsilikat, insbesondere der Klinoptilolith, in mechanisch modifizierter und aktivierter Form eingesetzt. In einer besonders bevorzugten Ausführungsform weisen die Partikel des Gerüstsilikats, insbesondere der Klinoptilolith, nach einer mechanischen Zerkleinerung eine Partikelgröße von 2 bis 16 µm auf. Die Partikelgröße kann beispielsweise mittels Siebanalyse bestimmt werden.

Synthetische Zeolithe werden aus anorganischen Vorläufern wie Kieselgel, Soda, Aluminiumoxid und anderen Salzen unter hydrothermalen Bedingungen auskristallisiert. Mehr als 150 verschiedene Zeolithtypen sind synthetisiert worden.

Es ist besonders bevorzugt, dass das Mittel als Gerüstsilikat ein natürliches Zeolith, insbesondere Klinoptilolith, umfasst.

Der Einsatz eines Gerüstsilikats, insbesondere von Klinoptilolith , in einem Mund- und/oder Zahnpflege- und/oder -reinigungsmittel kann beispielsweise Vorteile durch Bindung schwefelhaltiger bakterieller Stoffwechselprodukte, die bei einer entsprechenden Zusammensetzung der bakteriellen Plaque in der Mundhöhle auftreten und Ursache von Mundgeruch (Halitosis) sind, verleihen. Ebenso können Gerüstsilikat, insbesondere Klinoptilolith, dem Mund- und/oder Zahnpflege- und/oder -reinigungsmittel eine entzündungshemmende Wirkung durch Bindung von Histamin verleihen. Auch andere organische Verbindungen können durch das Gerüstsilikat gebunden und aus dem Mundraum transportiert werden.

Die Gerüstsilikate dienen auch als Poliermittel. Der Einsatz von Gerüstsilikaten, insbesondere von Klinoptilolith, mit Partikelgrößen in einem Bereich von 2 bis 16 µm ermöglicht eine schonende Behandlung der Zahnoberflächen.

Die Menge an dem mindestens einen Gerüstsilikat, vorzugsweise Klinoptilolith , in dem Mittel beträgt bevorzugt 10 bis 40 Gew.-%, mehr bevorzugt 12 bis 35 Gew.-% und ganz besonders bevorzugt 15 bis 30 Gew.-%, jeweils bezogen auf das Gesamtgewicht des Mittels.

Ein weiterer optionaler Inhaltsstoff des Mund- und/oder Zahnpflege- und/oder - reinigungsmittels ist ein natürliches Aroma. Ein natürliches Aroma kann einen Aromastoff oder ein Gemisch von Aromastoffen umfassen, das mittels geeigneter physikalischer, enzymatischer oder mikrobiologischer Verfahren aus Ausgangsstoffen pflanzlicher oder tierischer Herkunft gewonnen wird und mit in der Natur vorkommenden Aromastoffen chemisch identisch ist.

Das Mund- und/oder Zahnpflege- und/oder -reinigungsmittels kann ebenfalls optional ein Fluorid nicht-pflanzlichen Ursprungs enthalten. Das Fluorid nicht-pflanzlichen Ursprungs kann beispielsweise in Form anorganischer Fluoridsalze (zum Beispiel Natriumfluorid, Zinn(II)fluorid oder Natriummonofluorphosphat) oder in Form eines Aminfluorids wie Olaflur enthalten sein. Es kann bevorzugt sein, dass das Mund- und/oder Zahnpflege- und/oder -reinigungsmittel 500 bis 1600 ppm eines Fluorids nicht-pflanzlichen Ursprungs enthält. In einer ganz besonders bevorzugten Ausführungsform enthält das Mund- und/oder Zahnpflege- und/oder -reinigungsmittel kein Fluorid nicht-pflanzlichen Ursprungs.

Das Mittel kann in Form einer Zahncreme (Zahnpasta), eines Zahngels, eines Zahnpulvers, eines Mundwassers, einer Lippenpflegecreme, eines Mundpflegeserums oder eines Zahnpflegekaugummis vorliegen.

Ein weiterer Gegenstand der Anmeldung ist ein Verfahren zur Herstellung eines Mund- und/oder Zahnpflege- und/oder -reinigungsmittels, enthaltend in einem oral akzeptablen Träger mindestens drei Mineralstoffe pflanzlichen Ursprungs und mindestens einen Pflanzenextrakt, umfassend die Schritte:
- Herstellung des Pflanzenextrakts mittels Mazeration einer Mischung, umfassend - bezogen auf das Gesamtgewicht der Mischung - a) 1 bis 5 Gew,-% einer Pflanze und/oder eines Pflanzenbestandteils und b) 95 bis 99 Gew.-% einer Mischung aus Wasser und Glycerin, wobei das Mischungsverhältnis vorzugsweise im Bereich von 1 : 1 bis 1 : 7 liegt, und
- Mischen des erhaltenen Pflanzenextrakts mit mindestens drei Mineralstoffen pflanzlichen Ursprungs und einem oral akzeptablen Träger.

Die erfindungsgemäßen Mittel können in einem kosmetischen, nicht therapeutischen Verfahren zur Reinigung und Pflege des Mundes und/oder der Zähne bei gleichzeitiger Remineralisierung der Zähne, des Zahnfleisches, der Lippen und/oder der Mundschleimhaut eingesetzt werden.

Bezüglich bevorzugter Ausführungsformen der erfindungsgemäßen Verfahren gilt mutatis mutandis das zu den erfindungsgemäßen Mitteln Gesagte.

Besonders bevorzugte Mund- und/oder Zahnpflege- und/oder -reinigungsmittel A) bis Q) umfassen:
A) Mund- und/oder Zahnpflege- und/oder -reinigungsmittel, enthaltend in einem oral akzeptablen Träger mindestens acht Mineralstoffe pflanzlichen Ursprungs.
B) Mund- und/oder Zahnpflege- und/oder -reinigungsmittel, enthaltend in einem oral akzeptablen Träger mindestens acht Mineralstoffe pflanzlichen Ursprungs und mindestens vier Pflanzenextrakte.
C) Mund- und/oder Zahnpflege- und/oder -reinigungsmittel, enthaltend in einem oral akzeptablen Träger mindestens acht Mineralstoffe pflanzlichen Ursprungs, mindestens vier Pflanzenextrakte und mindestens einen Pflanzenbestandteil.
D) Mund- und/oder Zahnpflege- und/oder -reinigungsmittel, enthaltend in einem oral akzeptablen Träger mindestens acht Mineralstoffe pflanzlichen Ursprungs, mindestens vier Pflanzenextrakte und mindestens einen Pflanzenbestandteil enthält und wobei die Herstellung des Pflanzenextrakts mittels Mazeration einer Mischung, umfassend - bezogen auf das Gesamtgewicht der Mischung - a) 1 bis 5 Gew.-% einer Pflanze und/oder eines Pflanzenbestandteils und b) 95 bis 99 Gew.-% einer Mischung aus Wasser und Glycerin, wobei das Mischungsverhältnis vorzugsweise im Bereich von 1 : 1 bis 1 : 7 liegt, erfolgt.
E) Mund- und/oder Zahnpflege- und/oder -reinigungsmittel, enthaltend in einem oral akzeptablen Träger mindestens acht Mineralstoffe pflanzlichen Ursprungs, mindestens vier Pflanzenextrakte, mindestens einen Pflanzenbestandteil und mindestens ein Tonmineral.
F) Mund- und/oder Zahnpflege- und/oder -reinigungsmittel, enthaltend in einem oral akzeptablen Träger mindestens acht Mineralstoffe pflanzlichen Ursprungs, mindestens vier Pflanzenextrakte, mindestens einen Pflanzenbestandteil, mindestens ein pflanzliches Öl und mindestens ein Tonmineral.
G) Mund- und/oder Zahnpflege- und/oder -reinigungsmittel, enthaltend in einem oral akzeptablen Träger mindestens acht Mineralstoffe pflanzlichen Ursprungs, mindestens vier Pflanzenextrakte, mindestens einen Pflanzenbestandteil und mindestens ein Tonmineral, die Herstellung des Pflanzenextrakts mittels Mazeration einer Mischung, umfassend - bezogen auf das Gesamtgewicht der Mischung - a) 1 bis 5 Gew.-% einer Pflanze und/oder eines Pflanzenbestandteils und b) 95 bis 99 Gew.-% einer Mischung aus Wasser und Glycerin, wobei das Mischungsverhältnis vorzugsweise im Bereich von 1 : 1 bis 1 : 7 liegt, erfolgt.
H) Mund- und/oder Zahnpflege- und/oder -reinigungsmittel, enthaltend in einem oral akzeptablen Träger mindestens acht Mineralstoffe pflanzlichen Ursprungs, mindestens vier Pflanzenextrakte, umfassend Löwenzahn-Extrakt (Taraxacum officinale), Galgant-Extrakt (Alpinia officinarum), Schafgarben-Extrakt (Achillea millefolium) und Oregano-Extrakt (Origanum vulgare), und mindestens einen Pflanzenbestandteil, umfassend zerkleinerte Bertramwurzel.
I) Mund- und/oder Zahnpflege- und/oder -reinigungsmittel, enthaltend in einem oral akzeptablen Träger mindestens acht Mineralstoffe pflanzlichen Ursprungs, mindestens vier Pflanzenextrakte, umfassend Löwenzahn-Extrakt (Taraxacum officinale), Galgant-Extrakt (Alpinia officinarum), Schafgarben-Extrakt (Achillea millefolium) und Oregano-Extrakt (Origanum vulgare), mindestens einen Pflanzenbestandteil, umfassend zerkleinerte Bertramwurzel, und ein Tonmineral, welches Illit, Kaolin und/oder Montmorillonit.
J) Mund- und/oder Zahnpflege- und/oder -reinigungsmittel, enthaltend in einem oral akzeptablen Träger mindestens acht Mineralstoffe pflanzlichen Ursprungs, mindestens vier Pflanzenextrakte, umfassend Löwenzahn-Extrakt (Taraxacum officinale), Galgant-Extrakt (Alpinia officinarum), Schafgarben-Extrakt (Achillea millefolium) und Oregano-Extrakt (Origanum vulgare), mindestens einen Pflanzenbestandteil, umfassend zerkleinerte Bertramwurzel, und ein Tonmineral, welches Illit, Kaolin und/oder Montmorillonit enthält, wobei die Herstellung des Pflanzenextrakts mittels Mazeration einer Mischung, umfassend - bezogen auf das Gesamtgewicht der Mischung - a) 1 bis 5 Gew.-% einer Pflanze und/oder eines Pflanzenbestandteils und b) 95 bis 99 Gew.-% einer Mischung aus Wasser und Glycerin, wobei das Mischungsverhältnis vorzugsweise im Bereich von 1 : 1 bis 1 : 7 liegt, erfolgt.
K) Mund- und/oder Zahnpflege- und/oder -reinigungsmittel, enthaltend in einem oral akzeptablen Träger mindestens acht Mineralstoffe pflanzlichen Ursprungs und ein Gerüstsilikat.
L) Mund- und/oder Zahnpflege- und/oder -reinigungsmittel, enthaltend in einem oral akzeptablen Träger mindestens acht Mineralstoffe pflanzlichen Ursprungs, ein Gerüstsilikat und mindestens vier Pflanzenextrakte.
M) Mund- und/oder Zahnpflege- und/oder -reinigungsmittel, enthaltend in einem oral akzeptablen Träger mindestens acht Mineralstoffe pflanzlichen Ursprungs, ein Gerüstsilikat, mindestens vier Pflanzenextrakte und mindestens einen Pflanzenbestandteil.
N) Mund- und/oder Zahnpflege- und/oder -reinigungsmittel, enthaltend in einem oral akzeptablen Träger mindestens acht Mineralstoffe pflanzlichen Ursprungs, ein Gerüstsilikat, mindestens vier Pflanzenextrakte, mindestens einen Pflanzenbestandteil und mindestens ein Tonmineral.
O) Mund- und/oder Zahnpflege- und/oder -reinigungsmittel, enthaltend in einem oral akzeptablen Träger mindestens acht Mineralstoffe pflanzlichen Ursprungs, ein Gerüstsilikat, mindestens vier Pflanzenextrakte, mindestens einen Pflanzenbestandteil und mindestens ein pflanzliches Öl.
P) Mund- und/oder Zahnpflege- und/oder -reinigungsmittel, enthaltend in einem oral akzeptablen Träger mindestens acht Mineralstoffe pflanzlichen Ursprungs, ein Gerüstsilikat, mindestens vier Pflanzenextrakte, umfassend Löwenzahn-Extrakt (Taraxacum officinale), Galgant-Extrakt (Alpinia officinarum), Schafgarben-Extrakt (Achillea millefolium) und Oregano-Extrakt (Origanum vulgare), und mindestens einen Pflanzenbestandteil, umfassend zerkleinerte Bertramwurzel.
Q) Mund- und/oder Zahnpflege- und/oder -reinigungsmittel, enthaltend in einem oral akzeptablen Träger mindestens acht Mineralstoffe pflanzlichen Ursprungs, ein Gerüstsilikat, umfassend Klinoptilolith, mindestens vier Pflanzenextrakte, umfassend Löwenzahn-Extrakt (Taraxacum officinale), Galgant-Extrakt (Alpinia officinarum), Schafgarben-Extrakt (Achillea millefolium) und Oregano-Extrakt (Origanum vulgare) und mindestens einen Pflanzenbestandteil, umfassend zerkleinerte Bertramwurzel.

### Beispiel

**Tabelle 1: Zusammensetzung eines erfindungsgemäßen Mund- und/oder Zahnpflege- und/oder -reinigungsmittels (Angabe der Menge in Gew.-%)**

| Inhaltsstoff | A | B |
|---|---|---|
| Mineralstoffe pflanzlichen Ursprungs (u.a. Calcium (Ca), Chrom (Cr), Kupfer (Cu), Eisen (Fe), Magnesium (Mg), Mangan (Mn), Selen (Se), Zink (Zn)) in Wasser | 6 | 6 |
| Glycerin/Wasser (1,2 : 1)-Extrakt von Löwenzahn (komplette Pflanze) | 3 | 3 |
| Glycerin/Wasser (1,2 : 1)-Extrakt von Galgant (Wurzelstock) | 3 | 3 |
| Glycerin/Wasser (1,2 : 1)-Extrakt von Schafgarbe (Kraut) | 3 | 3 |
| Glycerin/Wasser (7 : 1)-Extrakt von Oregano (Kraut) | 3 | 3 |
| Bertramwurzelpulver | 0,1 | 0,1 |
| Grüne Tonerde (INCI: Illite, Kaolin, Montmorillonite) | 20 | - |
| Kokosöl | 5 | 5 |
| Xanthan | 1,8 | 1,8 |
| Klinoptilolith (ø 6 µm) | - | 20 |
| Glycerin (86,5 %) | ad 100 | ad 100 |

Zur Herstellung des Mund- und/oder Zahnpflege- und/oder -reinigungsmittels A wurde die grüne Tonerde mit dem Kokosöl erwärmt. Parallel wurde zunächst Xanthan mit den Pflanzenextrakten und der wässrigen Mineralstoff-Lösung intensiv gemischt. Anschließend wurden das Glycerin und die Tonmischung zugegeben. Am Ende wurde die zerkleinerte Bertramwurzel unter Rühren zugegeben.

Zur Herstellung des Mund- und/oder Zahnpflege- und/oder -reinigungsmittels B wurde Klinoptilolith mit dem Kokosöl erwärmt. Parallel wurde zunächst Xanthan mit den Pflanzenextrakten und der wässrigen Mineralstoff-Lösung intensiv gemischt. Anschließend wurden das Glycerin und die Klinoptilolih-Kokosöl-Mischung zugegeben. Am Ende wurde die zerkleinerte Bertramwurzel unter Rühren zugegeben.

Die Mund- und/oder Zahnpflege- und/oder -reinigungsmittel A und B wiesen eine hohe remineralisierende Wirkung auf.

## Patentansprüche

1. Mund- und/oder Zahnpflege- und/oder -reinigungsmittel, enthaltend in einem oral akzeptablen Träger mindestens drei Mineralstoffe pflanzlichen Ursprungs.

2. Mund- und/oder Zahnpflege- und/oder -reinigungsmittel gemäß Anspruch 1, **dadurch gekennzeichnet, dass** das Mittel mindestens fünf, mehr bevorzugt mindestens acht Mineralstoffe pflanzlichen Ursprungs enthält.

3. Mund- und/oder Zahnpflege- und/oder -reinigungsmittel gemäß einem der Ansprüche 1 oder 2, **dadurch gekennzeichnet, dass** der/die Mineralstoff(e) pflanzlichen Ursprungs ausgewählt ist/sind aus der Gruppe bestehend aus Calcium (Ca), Chrom (Cr), Kupfer (Cu), Eisen (Fe), Magnesium (Mg), Mangan (Mn), Selen (Se), Zink (Zn) und Mischungen daraus.

4. Mund- und/oder Zahnpflege- und/oder -reinigungsmittel gemäß einem der Ansprüche 1 bis 3, **dadurch gekennzeichnet, dass** das Mittel ferner ein pflanzliches Öl, vorzugsweise Kokosöl, enthält.

5. Mund- und/oder Zahnpflege- und/oder -reinigungsmittel gemäß einem der Ansprüche 1 bis 4, **dadurch gekennzeichnet, dass** das Mittel ferner mindestens einen Pflanzenextrakt enthält.

6. Mund- und/oder Zahnpflege- und/oder -reinigungsmittel gemäß Anspruch 5, **dadurch gekennzeichnet, dass** der Pflanzenextrakt ausgewählt ist aus der Gruppe bestehend aus Löwenzahn-Extrakt (Taraxacum officinale), Galgant-Extrakt (Alpinia officinarum), Schafgarben-Extrakt (Achillea millefolium), Oregano-Extrakt (Origanum vulgare) und Mischungen daraus.

7. Mund- und/oder Zahnpflege- und/oder -reinigungsmittel gemäß einem der Ansprüche 1 bis 6, **dadurch gekennzeichnet, dass** das Mittel ferner mindestens eine Pflanze und/oder einen Pflanzenbestandteil in zerkleinerter Form enthält.

8. Mund- und/oder Zahnpflege- und/oder -reinigungsmittel gemäß einem der Ansprüche 1 bis 7, **dadurch gekennzeichnet, dass** das Mittel ferner mindestens ein Tonmineral enthält.

9. Mund- und/oder Zahnpflege- und/oder -reinigungsmittel gemäß einem der Ansprüche 1 bis 8, **dadurch gekennzeichnet, dass** das Mittel ferner mindestens ein Gerüstsilikat umfasst.

10. Mund- und/oder Zahnpflege- und/oder -reinigungsmittel gemäß Anspruch 9, **dadurch gekennzeichnet, dass** das mindestens eine Gerüstsilikat Klinoptilolith umfasst.

11. Mund- und/oder Zahnpflege- und/oder -reinigungsmittel gemäß Anspruch 7, **dadurch gekennzeichnet, dass** die mindestens eine Pflanze und/oder der mindestens eine Pflanzenbestandteil in zerkleinerter Form aus der Pflanzengattung Bertram (Anacyclus) stammt.

12. Mund und- und/oder Zahnpflege- und/oder -reinigungsmittel gemäß Anspruch 11, **dadurch gekennzeichnet, dass** die mindestens eine Pflanze und/oder der mindestens eine Pflanzenbestandteil in zerkleinerter Form ausgewählt ist aus der Gruppe bestehend aus Deutscher Bertram (Anacyclus officinarum), Römischer Bertram (Anacyclus pyrethrum) und Mischungen daraus.

13. Mund und- und/oder Zahnpflege- und/oder -reinigungsmittel gemäß Anspruch 11 oder 12, **dadurch gekennzeichnet, dass** die mindestens eine Pflanze und/oder der mindestens eine Pflanzenbestandteil in zerkleinerter Form zerkleinerte Bertramwurzel umfasst.

14. Verfahren zur Herstellung eines Mund- und/oder Zahnpflege- und/oder -reinigungsmittels, enthaltend in einem oral akzeptablen Träger mindestens drei Mineralstoffe pflanzlichen Ursprungs und mindestens einen Pflanzenextrakt, umfassend die Schritte: Herstellung des Pflanzenextrakts mittels Mazeration einer Mischung, umfassend - bezogen auf das Gesamtgewicht der Mischung - a) 1 bis 5 Gew.-% einer Pflanze und/oder eines Pflanzenbestandteils und b) 95 bis 99 Gew.-% einer Mischung aus Wasser und Glycerin, wobei das Mischungsverhältnis vorzugsweise im Bereich von 1 : 1 bis 1 : 7 liegt, und Mischen des erhaltenen Pflanzenextrakts mit mindestens drei Mineralstoffen pflanzlichen Ursprungs und einem oral akzeptablen Träger.

15. Verfahren zur Reinigung und/oder Pflege des Mundes und/oder der Zähne bei gleichzeitiger Remineralisierung der Zähne, des Zahnfleisches, der Lippen und/oder der Mundschleimhaut, bei dem ein Mund- und/oder Zahnpflege- und/oder -reinigungsmittel gemäß einem der Ansprüche 1 bis 13 oder hergestellt nach Anspruch 14 eingesetzt wird.
